(19) Europäisches Patentamt
European Patent Office
Office européen des brevets

(11) Veröffentlichungsnummer: **0 031 958**
**B1**

(12) **EUROPÄISCHE PATENTSCHRIFT**

(45) Veröffentlichungstag der Patentschrift: **28.08.85**

(21) Anmeldenummer: **80108217.3**

(22) Anmeldetag: **27.12.80**

(51) Int. Cl.$^4$: **C 08 G 63/68,** C 08 G 63/62, C 08 G 63/66, C 07 C 149/36

# (54) Flammwidrige Polycarbonate mit verbesserter kritischer Dicke und Verfahren zu ihrer Herstellung.

(30) Priorität: **07.01.80 US 110273**

(43) Veröffentlichungstag der Anmeldung: **15.07.81 Patentblatt 81/28**

(45) Bekanntmachung des Hinweises auf die Patenterteilung: **28.08.85 Patentblatt 85/35**

(84) Benannte Vertragsstaaten: **DE FR GB IT NL**

(56) Entgegenhaltungen:
**EP-A-0 001 096**
**US-A-3 912 687**
**US-A-4 043 980**

**PATENTS ABSTRACTS OF JAPAN, Band 1, Nr. 29, 28. März 1977, Seite 1623C76**

**CHEMICAL ABSTRACTS, Band 83, Nr. 24, 15. Dezember 1975, Seite 57, Spalte 1, Abstract Nr. 194468r, Columbus, Ohio, US, I. OHMAE et al.: "Fireproofing agents for thermoplastics"**

(73) Patentinhaber: **Mobay Chemical Corporation**
**Penn Lincoln Parkway West**
**Pittsburgh, Pennsylvania 15205 (US)**

(72) Erfinder: **Krishnan, Sivaram**
**Achsenstrasse 12**
**D-4130 Moers (DE)**
Erfinder: **Baron, Arthur L.**
**211 East Thistle Court**
**New Martinsville, W.VA 26155 (US)**

(74) Vertreter: **Gremm, Joachim, Dr. et al**
**Bayer AG c/o Zentralbereich Patente, Marken und Lizenzen**
**D-5090 Leverkusen 1, Bayerwerk (DE)**

**EP 0 031 958 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind Copolycarbonate aus Diphenolen der Formel I

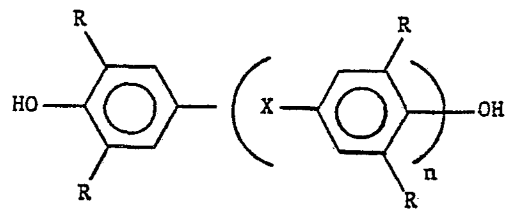

(I)

worin

R H oder $CH_3$ ist,

X $C_2$—$C_5$-alkylen, $C_1$—$C_5$-Alkyliden, —O, —$SO_2$— oder eine Einfachbindung ist und

n 1 oder 0 ist,

mit dem Diphenol der Formel II

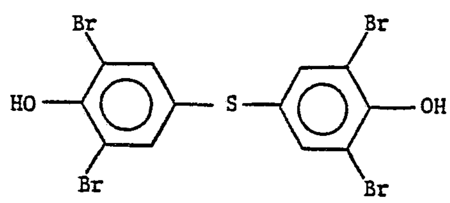

(II)

Die erfindungsgemäßen Copolycarbonate haben gute flammwidrige Eigenschaften und eine verbesserte kritische Dicke verglichen mit Copolycarbonaten auf Basis halogenfreier Thio-bisphenole und anderer halogenierter Bisphenole.

Halogenhaltige Polycarbonate auf Basis von Thio-diphenolen sind aus den US-Patenten Nr. 41 74 359 (& EP—A—1096) (Mo 1768—US—CIP) und Nr. 40 43 980 (Mo—1609—US) bekannt. Dort wird jedoch das Halogen nicht über die Thiodiphenolkomponente eingeführt.

Halogenierte Polycarbonate auf Basis schwefelhaltiger Diphenole sind auch aus der US—PS 3 912 688 bekannt. Das Halogen wird jedoch auch hier nicht über die schwefelhaltigen Diphenole in die Polycarbonate eingebracht.

Halogenierte Copolycarbonate aus tetrahalogenierten Thiphenolen sind aus dem US—PS 3 912 687 bekannt. Es werden dort jedoch mindestens 50 Mol-% an halogenierten Diphenolen eingesetzt. Derartige Produkte haben jedoch schlechte mechanische Eigenschaften, wie beispielsweise Kerbschlagzähigkeit, Thermostabilität und Reißspannung. Aus der US-Patentschrift 3 398 212 sind Copolycarbonate aus Thiodiphenolen (2 bis 50 Mol-%), Diphenolen mit geminalen polycyclischen Brückengliedern (mindestens 10 Mol-%) und gegebenenfalls auch anderen Diphenolen beschrieben.

Die Copolycarbonate können auch halogeniert sein. Das Halogen kann auch über das Thiodiphenol eingeführt werden, wobei allerdings nur ein chloriertes Thiodiphenol aufgeführt ist. (Siehe Spalte 6, Zeile 22 sowie Beispiel 8b der US—PS 3 398 212). Über die kritische Dicke derartiger Copolycarbonate wird in US—PS 3 398 212 nichts ausgeführt.

Aus der japanischen Offenlegungsschrift Nr. 67850/75 ist bekannt, niedermolekulare Polycarbonate (Polymerisationsgrad 2 bis 10) aus Tetrachlor- oder Tetrabromthiodiphenolen thermoplastichen Kunststoffen beispielsweise Bisphenol-A-Polycarbonaten, als Flammschutzmittel in Mengen von 1 bis 50 Gew.-% bezogen auf thermoplastischen Kunststoffen, zuzusetzen.

Mischungen aus thermoplastischem Bisphenol-A-Polycarbonat und niedermolekularen Tetrabromthiodiphenol-Polycarbonaten zeigen jedoch Trübung. Über die kritische Dicke derartiger Mischungen wird in der japanischen Offenlegungsschrift nichts ausgeführt.

Copolycarbonate im Sinne der vorliegenden Erfindung sind somit solche mit Struktureinheiten

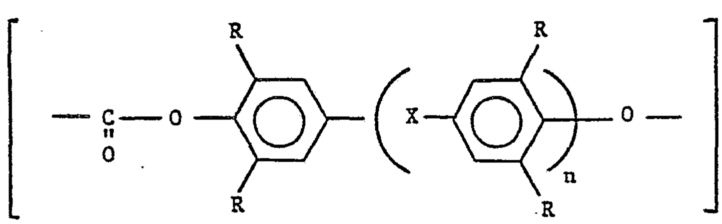

(Ia)

worin

R, X und n die für die Formel (I) genannte Bedeutung haben,

und mit Struktureinheiten

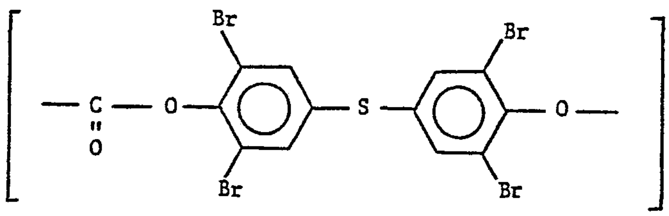

(IIa)

wobei zwischen 2 und 20 Gew.-%, vorzugsweise zwischen 2 und 10 Gew.-%, an Struktureinheiten der Formel IIa bezogen auf die Summe der Struktureinheiten Ia + IIa, vorliegen, entsprechend etwa zwischen 1 und 10 Mol-%, vorzugsweise zwischen 1 und 5 Mol-% an IIa, bezogen auf die Summe Ia + IIa.

Die erfindungsgemäßen Copolycarbonate haben mittlere Gewichtsmittelmolekulargewichte $\overline{M}n$ zwischen 10 000 und 200 000 (gemessen in Ethylendichlorid) und vorzugsweise Schmelzindices von 1 bis 24 g/10 Minuten bei 300°C und insbesondere von 1 bis 15 g/10 Minuten bei 300°C entsprechend ASTM 1238.

Die erfindungsgemäßen Copolycarbonate können nach den bekannten Verfahren für die Polycarbonatherstellung aus den Diphenolen der Formeln I und II synthetisiert werden, wobei jeweils von 2 bis 20 Gew.-%, vorzugsweise von 2 bis 10 Gew.-%, bezogen jeweils auf die Gewichtsumme der eingesetzten Diphenole I + II, an Diphenolen II verwendet werden, bzw. jeweils von 1 bis 10 Mol-%, vorzugsweise von 1 bis 5 Mol-%, bezogen jeweils auf die Gesamtmolmenge an eingesetzten Diphenolen I + II, verwendet werden.

Das bevorzugte Herstellungsverfahren ist das Zweiphasengrenzflächenverfahren, wobei das Diphenol II bevorzugt als etwa 10 %ige wäßrige Bis-Natriumdiphenolat-Lösung eingesetzt wird. Als Kettenabbrecher dienen die üblichen monofunktionellen Ver bindungen wie Phenol, Propyl-phenole, Butyl-phenole, Isopropyl-phenole, insbensondere jedoch Phenol und -tert.-Butylphenol.

Als Katalysatoren dienen die bekannten tertiären Amine, quartären Ammoniumsalze, Phosphoniumsalze und Arsoniumsalze. Als Lösungsmittel sind insbesondere $CH_2Cl_2$ und Chlorbenzol geeignet.

Die Reaktionstemperaturen des Zweiphasengrenzflächenverfahrens liegen zwischen −20°C und 150°C, vorzugsweise zwischen 0°C und 100°C.

Entsprechend dem Polycarbonat-Herstellungsverfahren in homogener Phase werden die in inerten Lösungsmitteln gelösten Reaktionspartner in Gegenwart äquivalenter Mengen tertiärer Aminbasen, wie beispielsweise N,N-Dimethylanilin, N,N-Dimethyl-cyclohexylamin, oder vorzugsweise Pyridin polykondensiert.

Entsprechend dem Umesterungsverfahren werden die erfindungsgemäß einzusetzenden Diphenole mit Diarylcarbonaten zu den erfindungsgemäßen Polycarbonaten umgeestert.

Weitere Einzelheiten für die Herstellung der erfindungsgemäßen Copolycarbonate sind beispeielsweise in den US—Patenten 3 028 365, 3 062 781 und 3 912 688 sowie in der Monographie "H. Schnell, Chemistry and Physics of Polycarbonates" Interscience Publishers, New York, 1964 enthalten.

Geeignete Diphenole der Formel I sind beispielsweise Bis-(4-hydroxyphenyl)-methan, 2,2-Bis-(4-hydroxyphenyl)-propan, 2,2-Bis-(4-hydroxy-3,5-dimethylphenyl)-propan, Bis(4-hydroxy-3,5-dimethylphenyl)-methan, 4,4'-Dihydroxydiphenyl, 4,4'-Dihydroxydiphenyl-ether, 4,4'Dihydroxydiphenylsulfon, Hydrochinon, 2,2-Bis-(4-hydroxy-phenyl)-butan, 2,2-Bis-(4-hydroxy-phenyl)-pentan, 3,3-Bis-(4-hydroxy-phenyl)-pentan, 2,4-Bis-(4-hydroxy-phenyl)-2-methyl-butan und 2,4-Bis-(3,5-dimethyl-4-hydroxy-phenyl)-2-methyl-butan.

Das Bis-(4-hydroxy-3,5-dibrom-phenyl)-sulfid der Formel II kann nach üblichen Methoden hergestellt werden, beispielsweise durch Bromierung von Bis-(4-hydroxy-phenyl)-sulfid mit Brom in Eisessig.

Ein besonders günstiges Verfahren zur Herstellung eines sehr reinen Tetrabrom-thioethers der Formel II ist folgendes:

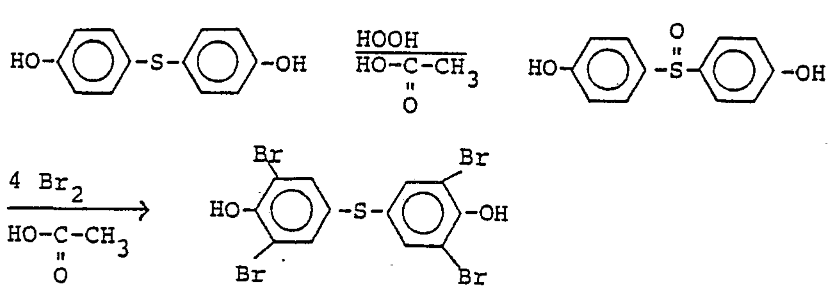


Bei diesem Verfahren zur Herstellung von Bis-(4-hydroxy-3,5-dibrom-phenyl)-sulfid oxydiert man Bis-(4-hydroxyphenyl)-sulfid mit $H_2O_2$ in Eisessig bei Temperaturen von 80—90°C zum Bis-(4-hydroxy-phenyl)-sulfoxid und setzt dieses anschließend mit Brom in Eisessig bei Temperaturen von 80°C—90°C zum Bis-(4-hydroxy-3,5-dibrom-phenyl)-sulfid.

Die Ausbeuten an Bis-(4-hydroxy-3,5-dibrom-phenyl)-sulfid sind hierbei 98% der Theorie, das Produkt fällt in einer Reinheit von 99% an.

Der Halogengehalt, insbesondere Bromgehalt, der erfindungsgemäßen Copolycarbonate soll vorzugsweise zwischen 3 und 10 Gew.-% liegen. Dies kann außer durch das Bis-(4-hydroxy-3,5-dibrom-phenyl)-sulfid auch dadurch erreicht werden, daß andere halogenierte Diphenole der Formel III

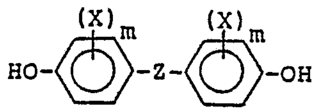

(III)

worin

Z 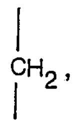 , 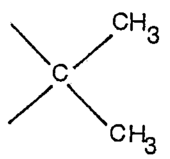 oder 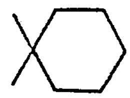 ,

m 1 oder 2 und

X Cl oder Br sind,

für die Polycarbonatherstellung mitverwendet werden.

Gegenstand der vorliegenden Erfindung sind somit auch Copolycarbonate mit Halogengehalten, vorzugsweise Bromgehalten, zwischen 3 und 10 Gew.-%, die dadurch gekennzeichnet, sind, daß sie Struktureinheiten der Formel Ia, 2 bis 20 Gew.-%, vorzugsweise 2 bis 10 Gew.-%, bezogen jeweils auf die Summe der Struktureinheiten Ia + IIa, Struktureinheiten IIa und Struktureinheiten der Formel IIIa

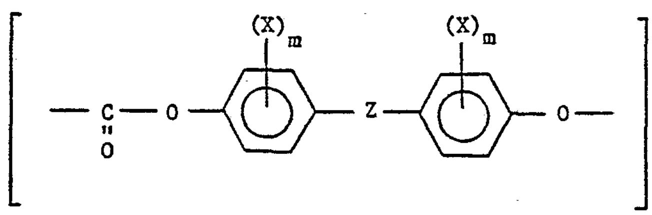

(IIIa)

worin

Z, X und m die für Formel III genannte Bedeutung haben,

in den Mengen enthalten, daß der Halogengehalt, vor zugsweise Bromgehalt, des Copolycarbonats zwischen 3 und 10 Gew.-% liegt.

Halogenierte Diphenole der Formel III sind beispielsweise 2,2-Bis-(3,5-dibrom-4-hydroxyphenyl)-propan, 2,2-Bis-(3,5-dichlor-4-hydroxyphenyl)-propan, 2,2-Bis(3-brom-4-hydroxyphenyl)-propan, 2,2-Bis-(3-chlor-4-hydroxyphenyl)-propan, Bis-(3,5-dibrom-4-hydroxyphenyl)-cyclohexan oder Bis-(3,5-dichlor-4-hydroxyphenyl)-methan.

Gegenstand der vorliegenden Erfindung ist außerdem ein Verfahren zur Herstellung der Copolycarbonate mit Halogengehalten, insbesondere Bromgehalten, von 3 bis 10 Gew.-%, bestehend aus den Struktureinheiten Ia + IIa + IIIa, dadurch gekennzeichnet, daß man Diphenole I, 2 bis 20 Gew.-%, vorzugsweise 2 bis 10 Gew.-%, bezogen auf die Summe der Diphenole I + II, Diphenole II und Diphenole III nach den bekannten Polycarbonatherstellungsverfahren umsetzt, wobei die Menge an Diphenol III so bemessen ist, daß der Halogengehalt, vorzugsweise Bromgehalt, des Copolycarbonats zwischen 3 und 10 Gew.-% liegt.

Die erfindungsgemäßen Copolycarbonate können in üblicher Weise gereinigt und isoliert und zu Formkörpern und Folien verarbeitet werden, beispielsweise via Extrusion oder Spiritzguß oder nach dem Gießverfahren.

Den erfindungsgemäßen Copolycarbonaten können die üblichen Stabilisatoren gegen Hitze, Feuchtigkeit und UV-Licht-Einwirkung einverleibt werden, ebenso Füllstoffe, faserartige Zusätze beispielsweise Glasfasern, oder auch Pigmente, wie dies aus der Polycarbonatchemie üblich ist.

Beispiel 1
(außerhalb des Anspruchsumfangs)

Herstellung des Bis(3,5-dibrom-4-hydroxyphenyl)-sulfids

a) In einem Reaktionsgefäß werden 21,8 g (0,1 Mol) 4,4'-Thio-diphenol und 300 ml Essigsäure bei Raumtemperatur vorgelegt. 64 g Brom (0,4 Mol) werden innerhalb von 70 Minuten hinzugefügt. Danach wird die Temperatur auf 65°C angehoben und nach 10 Minuten scheidet sich ein rotes Rohprodukt bei 60°C aus. Das Reaktionsgemisch wird langsam heller und nach weiteren 35 Minuten ist das Reaktionsge. misch hellrot. Nach weiteren 15 Minuten wird die Temperatur auf 75°C erhöht und 1,5 Stunden bei diesem Wert gehalten. Das Reaktionsprodukt wird danach abfiltriert und mit Wesser gewaschen. Der Schmelzpunkt ist 208—212°C.

b) Herstellung über das 4,4'-Bis-(hydroxyphenyl)-sulfoxid.

Eine Mischung von 109 g (0,5 Mol) 4,4-Thiobisphenol und 150 ml Essigsäure werden auf 80°C erhitzt bis das Thiobisphenol gelöst ist. Wasserstoffperoxid (34 g, 0,5 Mol 50% waßrige Lösung) wird tropfenweise innerhalb von 1 1/2 Stunden zugegeben, wobei die Temperatur konstant gehalten wird. Nachdem etwa die Hälfte des Wasserstoffperoxids zugegeben worden ist, fällt das Sulfoxyd aus. Nach der vollständigen Zugabe von Wasserstoffperoxid wird die Reaktionsmischung auf 90°C erwärmt, für 1 1/2 Stunden auf dieser Temperatur gehalten und 200 CC Wasser zugegeben. Die ausgefallenen farblosen Kristalle werden filtriert und gut mit Wasser gewaschen. Ausbeute: 106 g (90% der Theorie) Schmelzpunkt 194°C.

Das Sulfoxid wird in Bis(3,5-dibromo-4-hydroxyphenyl) Sulif nach der gleichen Vorschrift (a) umgesetzt. Die Ausbeute beträgt 98% und die Reinheit 99% (Dünnschicht Chromatographic bestimmt) Schmelzpunkt 212—213°C.

Beispiel 2

Ein Copolycarbonat wird hergestellt, indem man nach dem Zweiphasengrenzflächenverfahren die Bisnatriumsalze von 2,2-Bis-(4-hydroxyphenyl)-propan und Bis-(4-hydroxy-3,5-dibrophenyl)-sulfid umsetzt. Hierbei wird das Bis-natrium-Salz des Bis-(4-hydroxy-3,5-dibromphenyl)-sulfids als etwa 10%ige wäßrige Lösung eingespeist. Das Gewichtsverhältnis von Bisphenol A zu Bis-(4-hydroxy-3,5-dibromphenyl)-sulfid beträgt 91:9. Das erhaltene Polycarbonat hatte 4,62 Gew.-% Brom und 0,46 Gew.-% Schwefel. Die Einfriertemperatur (gemessen nach ASTM D 648) war 142°C bei 1820 kPa (264 psi) ASTM. Die relative Viskosität des Copolycarbonats η rel (gemessen in $CH_2Cl_2$ bei 20°C, C = 0,5 Gew.-%) beträgt 1.273.

Die Schmelzstabilität des Copolycarbonats bei 300°C war wie folgt:

5 Minuten Schmelzviskosität 600 Pa.s.
35 Minuten Schmelzviskosität 545 Pa.s.
65 Minuten Schmelzviskosität 540 Pa.s.

Bei 288°C hergestellte Spritzgußkörper besitzen eine Helligkeit von 87,55%, bei 343°C hergestellte Spritzgußkörper besitzen eine Helligkeit von 81,72%. Weitere physikalischen Eigenschaften enthält die Tabelle.

Beispiel 3
(Stand der Technik)

Ein Copolycarbonat wurde gemäß US—PS 4 043 980 (Mo-1609) aus 87,45 Gew.-% 2,2-Bis-(4-hydroxy-phenyl)-propan, 10,3 Gew.-% 2,2-Bis-(3,5-dibrom-4-hydroxyphenyl)-propan und 2,25 Gew.-% 4,4-Bishydroxyphenyl-sulfid hergestellt. Die physikalischen Eigenschaften enthält die Tabelle. Die relative Viskosität des Copolycarbonats η rel (gemessen in $CH_2Cl_2$ bei 20°C, C = 0,5 Gew.-%) beträgt 1.300.

Beispiel 4
(Stand der Technik)

Ein Copolycarbonat wurde aus 90 Gew.-% 2,2-Bis-(4-hydroxyphenyl)-propan und 10 Gew.-% 2,2-Bis-(3,5-dibrom-4-hydroxyphenyl)-propan synthetisiert. Die physikalischen Eigenschaften enthält die Tabelle. Die relative Viskosität des Copolycarbonats η rel (gemessen in $CH_2Cl_2$ bei 20°C, C = 0,5 Gew.-%) beträgt 1.303.

TABELLE

| Beispiel | $\eta$ rel | Kritische Dicke | Izod-Kerbschlagzähigkeit[1] kg-cm/cm 1/8" (3.18 mm) | 1/4" (6.35 mm) | UL-94 1/8" | Flamm-Test 1/16" | Schmelzindex (g/10 Minuten) |
|---|---|---|---|---|---|---|---|
| 2 | 1.273 | 3.99 mm | 79,7 | 10,23 | V-O | V-O[2] | 6,3 |
| 3 | 1.300 | 3.45 mm | 78,7 | 13,4 | V-O | V-O | 3,1 |
| 4 | 1.303 | 3.3 mm | 79,0 | 11,97 | V-O | V-2 | 2,9 |

1) Kerbschlagzähigkeit gemessen nach üblicher Vorschrift.

2) Drei von fünf Proben hatten V-O, die übrigen Proben hatten V-2.

Der UL-94-Flamm-Test wurde wie folgt durchgeführt:

Teststäbe aus Polycarbonate einer Abmessung von 1.27 mm × 12.7 mm × 1.6 mm wurden vertikal aufgehängt, 305 mm über eine Baumwolle.

Jeder Teststab wird einzeln entzündet zweimal im Abstand von 10 Sekunden und die Brandcharakteristik nach jeder Entzündung wird festgestellt und eingeordnet. Man benutzt einem Bunsenbrenner, um die Teststäbe anzuzünden mit einer blauen Flamme von 10 mm Länge von Erdgas, das einen Wärmegehalt von ungefähr 1000 BTU (Britische wärme Thermoeinheiten pro Kubicfuß) hat.

Die UL-94-V-O Klassifizierung liegt dann vor, wenn keiner der Teststäbe länger als 10 Sekunden nach jeder Beflammung mit der Bunsenbrennerflamme brennt; jeder Satz an Teststäben (Anzahl 5) insgesamt nicht mehr als 50 Sekunden bei der zweimaligen Beflammung nachbrennt; keiner der Testäbe vollständig bis zur Halterung, die sich am oberen Ende der Teststäbe befindet, abbrennt; keiner der Teststäbe durch brennende Tropfen oder Partikel die darunter liegende baumwolle entszündet; und keiner der Teststäbe länger als 30 Sekunden nach Entfernung der Flamme nachglüht.

Die anderen UL-94 Klassifizierungen beinhalten Teststäbe, welche weniger flammwidrig und weniger selbstverlöschend sind, und von denen flammende Tropfen oder Partikel abfallen. Derartige Klassifizierungen sind als V-1 bzw. V-2 ausgewiesen.

**Patentansprüche**

1. Copolycarbonate mit Molekulargewichten ($\overline{M}w$) von 10 000 bis 200 000 und Struktureinheiten der Formel Ia

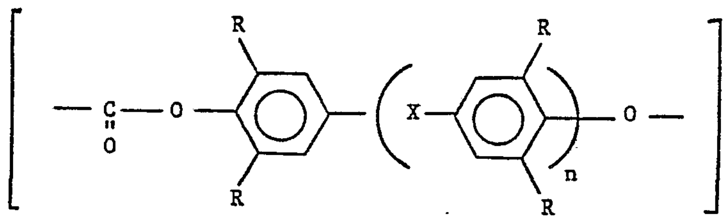

(Ia)

worin

R H oder $CH_3$ ist,

X $C_2$—$C_5$-alkylen, $C_1$—$C_5$-alkyliden, —O—, —$SO_2$— oder eine Einfachbindung ist und

n 1 oder 0 ist,

dadurch gekennzeichnet, daß sie zwischen 2 und 20 Gew.-%, bezogen auf die Summe der Struktureinheiten Ia + IIa, an Struktureinheiten IIa haben

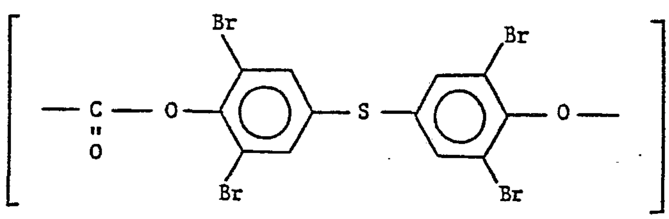

2. Copolycarbonate gemäß Anspruch 1, dadurch gekennzeichnet, daß sie 2 bis 10 Gew.-%, bezogen auf die Summe der Struktureinheiten Ia + IIa, an Struktureinheiten IIa haben.

3. Verfahren zur Herstellung der Copolycarbonate des Anspruchs 1, dadurch gekennzeichnet, daß man die Diphenole der Formeln I und II

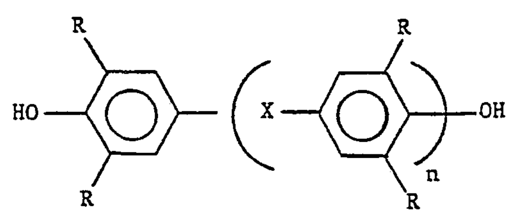

(I)

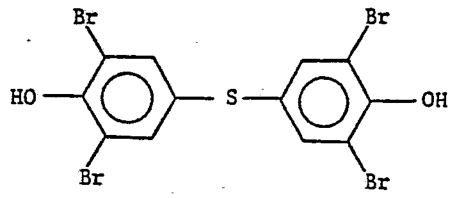

(II)

worin

R H oder $CH_3$ ist,

X $C_2$—$C_5$-alkylen, $C_1$—$C_5$-alkyliden, —O—, —$SO_2$— oder eine Einfachbindung ist und

n 1 oder 0 ist,

nach den bekannten Polycarbonatherstellungsverfahren polykondensiert, wobei jeweils von 2 bis 20 Gew.-%, bezogen auf die Gewichtssumme der eingesetzten Diphenole I + II, an Diphenolen II verwendet werden.

4. Verfahren zur Herstellung der Copolycarbonate des Anspruchs 2, dadurch gekennzeichnet, daß man gemäß Verfahren des Anspruchs 3 jeweils von 2 bis 10 Gew.-%, bezogen auf die Gewichtssumme der eingesetzten Diphenole I + II, an Diphenolen II verwendet werden.

5. Copolycarbonate gemäß Ansprüche 1 und 2, dadurch gekennzeichnet, daß sie außerdem Struktureinheiten der Formel IIIa enthalten

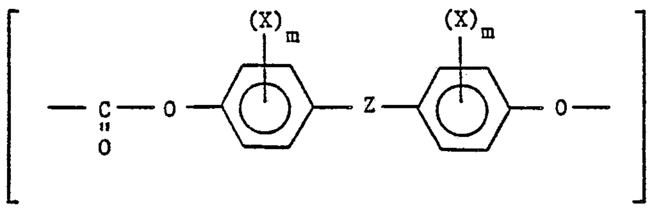

(IIIa)

worin

Z 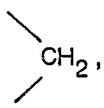 , 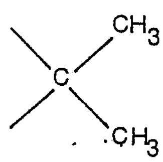 oder 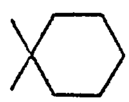 ,

m 1 oder 2 und

X Cl oder Br sind,

in den Mengen enthalten, daß der Halogengehalt des Copolycarbonats zwischen 3 und 10 Gew.-% lieft.

6. Verfahren zur Herstellung der Copolycarbonate des Anspruchs 5, dadurch gekennzeichnet, daß man Diphenole I, 2 bis 20 Gew.-%, vorzugsweise 2 bis 10 Gew.-%, bezogen auf die Summe der Diphenole I + II, Diphenole II und Diphenole III zu Copolycarbonaten umsetzt, wobei die Menge an Diphenol III so bemessen ist, daß der Halogengehalt des Copolycarbonats zwischen 3 und 10 Gew.-% liegt.

**Claims**

1. Copolycarbonates having molecular weights ($\overline{M}w$) of 10,000 to 200,000 and structural units of the formula Ia

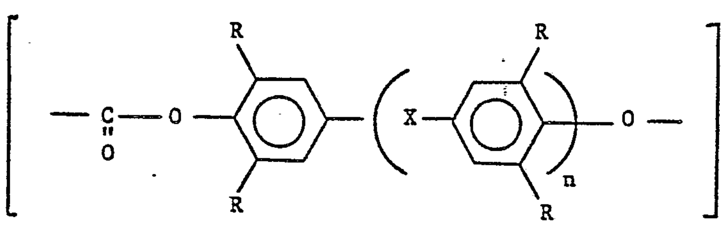

(Ia)

wherein

R is H or $CH_3$,

X is $C_2$—$C_5$-alkylene, $C_1$—$C_5$-alkylidene, —O—, —$SO_2$— or a single bond and

n is 1 or 0,

characterised in that they have between 2 and 20% by weight, based on the sum of the structural units Ia + IIa, of structural units IIa

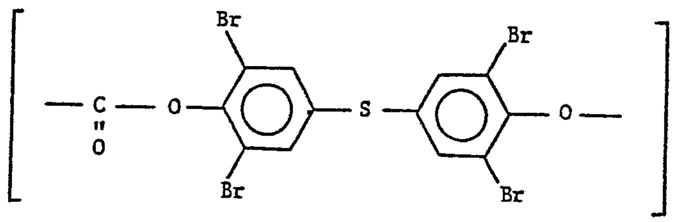

2. Copolycarbonates according to Claim 1, characterised in that they have 2 to 10% by weight, based on the sum of the structural units Ia + IIa, of structural units IIa.

3. Process for the production of the copolycarbonates of Claim 1, characterised in that the diphenols of the formulae I and II

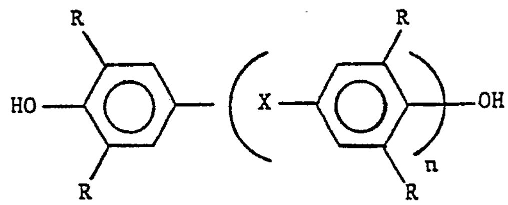

(I)

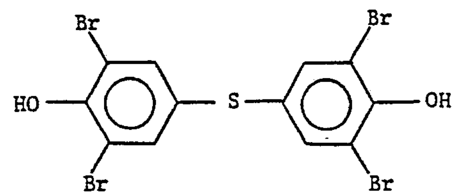

(II)

wherein

R is H or $CH_3$,

X is $C_2$—$C_5$-alkylene, $C_1$—$C_5$-alkylidene, —O—, —$SO_2$— or a single bond and

n is 1 or 0,

are polycondensed by the known methods for the production of polycarbonates, 2 to 20% by weight, based on the total weight of the diphenols I + II used, of diphenols II being used in each case.

4. Process for the production of the copolycarbonates of Claim 2, characterised in that, according to the process of Claim 3, 2 to 10% by weight, based on the total weight of the diphenols I + II used, of diphenols II are used in each case.

5. Copolycarbonates according to Claims 1 and 2, characterised in that they also contain structural units of the formula IIIa

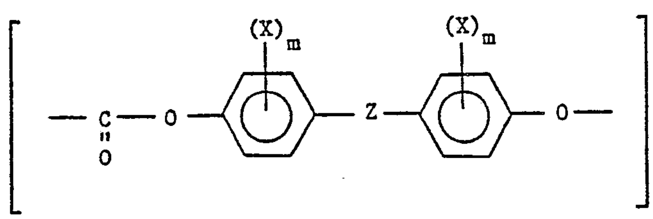

(IIIa)

wherein

Z is 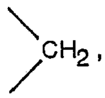 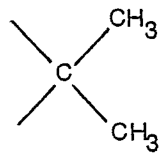 or 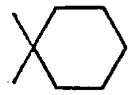 ,

m is 1 or 2 and

X is Cl or Br,

in such quantities that the halogen content of the copolycarbonate is between 3 and 10% by weight.

6. Process for the production of the copolycarbonates of Claim 5, characterised in that diphenols I, 2 to 20% by weight, preferably 2 to 10% by weight, based on the sum of diphenols I + II, of diphenols II and diphenols III are reacted to give copolycarbonates, the quantity of diphenol III being such that the halogen content of the copolycarbonate is between 3 and 10% by weight.

**Revendications**

1. Copolycarbonates ayant des poids moléculaires ($\overline{M}w$) de 10 000 à 200 000 et contenant des motifs de structure de formule Ia

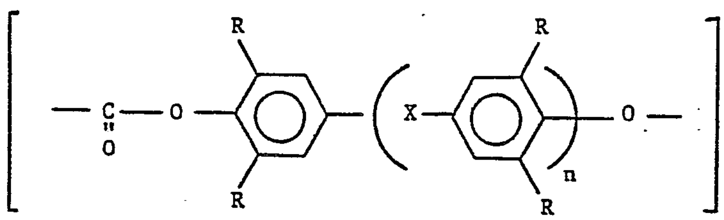

(Ia)

dans laquelle

R représente H ou $CH_3$,

X représente un groupe alkylène en $C_2$—$C_5$, alkylidène en $C_1C_5$, —O—, —$SO_2$— ou une liaison simple et n est égal à 1 ou 0,

caractérisés en ce qu'ils contiennent de 2 à 20% en poids, par rapport à a somme des motifs de structure Ia + IIa, de motifs de structure IIa

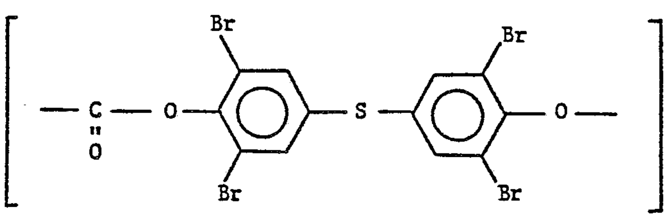

2. Copolycarbonates selon la revendication 1, caractérisés en ce qu'ils contiennent de 2 à 10% en poids, par rapport à la somme des motifs de structure Ia + IIa, de motifs de structure IIa.

3. Procédé de préparation des copolycarbonates de la revendication 1, caractérisé en ce que l'on polycondense, par les procédés connus de préparation des polycarbonates, les diphénols de formules I et II

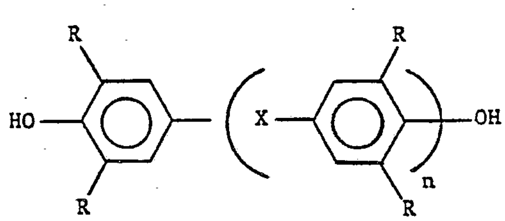

(I)

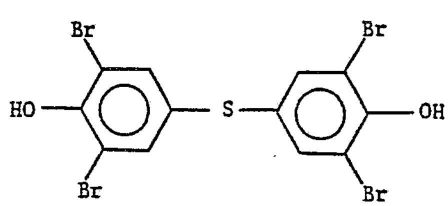

(II)

dans lesquelles

R représente H ou $CH_3$,

X représente un groupe alkylène en $C_2$—$C_5$, alkylidène en $C_1$—$C_5$, —O—, —$SO_2$— ou une liaison simple et

n est égal à 1 ou 0,

en utilisant dans chaque cas de 2 à 20% en poids, par rapport à la somme totale des diphénols I + II mis en oeuvre, de diphénols II.

4. Procédé de préparation des copolycarbonates de la revendication 2, caractérisé en ce que l'on utilise selon le procédé de la revendication 3 dans chaque cas 2 à 10% en poids, par rapport à la somme totale des diphénols I + II mis en oeuvre, de diphénols II.

5. Copolycarbonates selon les revendications 1 et 2, caractérisés en ce qu'ils contiennent en outre des motifs de structure de formule IIIa

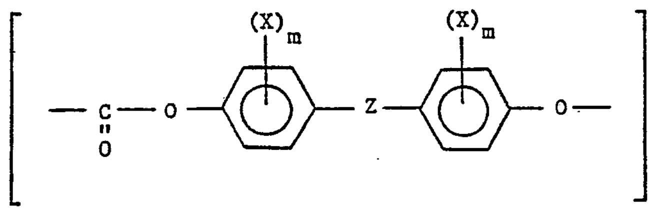

(IIIa)

dans laquelle

Z représente 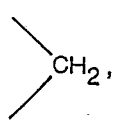, 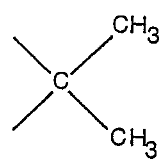 ou 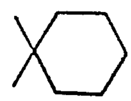

m est égal à 1 ou 2 et

X représente Cl ou Br,

en quantité telle que la teneur en halogène du copolycarbonate soit de 3 à 10% en poids.

Procédé de préparation des copolycarbonates de la revendication 5, caractérisé en ce que l'on convertit les diphénols I, 2 à 20% en poids, de préférence 2 à 10% en poids, par rapport à la somme des bis-phénols I + II, des diphénols II et les diphénols III en copolycarbonates, la quantité du diphénol III étant réglée de manière que la teneur en halogène du copolycarbonate soit de 3 à 10/ en poids.